# EUROPEAN PATENT APPLICATION

(11) **EP 3 881 842 A1**
(43) Date of publication of application: **22.09.2021**
(21) Application number: 20164331.9
(22) Date of filing: 19.03.2020
(51) Int. Cl.: A61K 31/196, A61P 25/00, G01N 33/50

(54) **COMPOSITIONS FOR THE TREATMENT OF AUTISM SPECTRUM DISORDER**

(71) Applicant: Stalicla S.A., 1202 Geneva (CH)
(72) Inventor: BURHAM, Lyn, 1202 Geneva (CH)
(74) Representative: Stolmár & Partner Intellectual Property GmbH

(57) **Abstract**

The present invention relates to pharmaceutical compositions for the treatment of a subtype of autism spectrum disorder (ASD) patients. Likewise, the present invention relates to methods of diagnosing a subtype of ASD and methods for identifying patients responding a specific treatment of ASD.

## Description

### Field of the invention

The present invention relates to pharmaceutical compositions for the treatment of a subtype of autism spectrum disorder (ASD) patients. Likewise, the present invention relates to methods of diagnosing a subtype of ASD and methods for identifying patients responding a specific treatment of ASD.

### Background of the invention

Autism spectrum disorder (ASD) is one of the most prevalent and disabling neurodevelopmental disorders (NDD). The prevalence of ASD is currently estimated at 1% in the world population and 1 in 59 school-aged children in the US (1 in 37 boys and 1 in 151 girls) (Baio et al. Prevalence of Autism Spectrum Disorder Among Children Aged 8 Years - Autism and Developmental Disabilities Monitoring Network, 11 Sites, United States, 2014. MMWR Surveill Summ. 2018 Apr 27;67(6):1-23). ASD is still considered a single diagnostic entity whose diagnosis remains based on observation of atypical behaviors characterized by deficits in 1) social interactions and communication, including deficits in social-emotional reciprocity; nonverbal communicative behaviors used for social interaction, and in developing, maintaining, and understanding relationships; 2) at least 4 subdomains of restricted or repetitive behaviors, including stereotyped or repetitive motor movements, insistence on sameness or inflexible adherence to routines, highly restricted and fixated interests, hyper- or hyporeactivity to sensory input, or unusual interest in the sensory aspects of the environment (Baird G, et al. Neurodevelopmental disorders. American Psychiatric Association. Diagnostic and Statistical Manual of Mental Disorders-Fifth Edition (DSM-5). Washington, D.C.: American Psychiatric Publishing, 2013: p. 31-86).

Autism is known has a "spectrum disorder" because of the wide heterogeneity in etiology, phenotype, severity and outcome. These factors contribute to a clinical heterogeneity that manifest as diverse deficits or impairments in behavioral features and communicative functioning. This marked heterogeneity of the condition has led to suggestions that rather than a single disorder, ASD, or "the autisms", encompass multiple etiologies and distinct clinical entities. Therefore, the current behavioral-based approaches to diagnose ASD patients do not provide an efficient and precise classification in terms of genetic alterations and disrupted molecular pathways.

Characterizing the heterogeneity of ASD is further complicated by the occurrence of psychopathologic and medical comorbidities. Anxiety, depression, ADHD, intellectual disability are over-represented in patients with autism suggesting that the underlying biological mechanisms are not independent (Gillberg et al. The ESSENCE in child psychiatry: Early Symptomatic Syndromes Eliciting Neurodevelopmental Clinical Examinations.Res Dev Disabil. 2010 Nov-Dec;31(6):1543-51; Hansen et al., Neurodevelopmental disorders: prevalence and comorbidity in children referred to mental health services. Nord J Psychiatry. 2018 May;72(4):285-291). For example, 30-50% of patients diagnosed with ASD also present some ADHD symptoms, and similarly, around 70% of ADHD patients display ASD core symptoms (Leitner et al. The co-occurrence of autism and attention deficit hyperactivity disorder in children - what do we know? Front Hum Neurosci. 2014 Apr 29;8:268). Furthermore, neurodevelopmental disorders (NDDs), including ASD, not only share some (core) symptoms, but also neuropathology and genetics etiologies to such an extent that NDD could actually be considered as a broader neurodevelopmental spectrum, rather than distinct disorders (Kushki et al., Examining overlap and homogeneity in ASD, ADHD, and OCD: a data-driven, diagnosis-agnostic approach. Transl Psychiatry 9, 318, 2019; Kern et al. Shared Brain Connectivity Issues, Symptoms, and Comorbidities in Autism Spectrum Disorder, Attention Deficit/Hyperactivity Disorder, and Tourette Syndrome. Brain Connect. 2015 Aug;5(6):321-35), related to neuronal insults, such as neuroinflammation driven by an excess of proinflammatory cytokines, excitotoxicity, sustained microglial activation, or oxidative stress. Therefore, these disorders may share a similar etiology.

Medical comorbidities of ASD include seizures, sleep difficulties, gastrointestinal disorders, mitochondrial dysfunction, and immune system abnormalities. The presence of one or more of these comorbidities is likely to be associated with more severe autism-related symptoms. For example, 11%-39% of individuals with ASD also have epilepsy, and these individuals are more likely to have severe social impairments than those diagnosed with ASD only. Comorbid sleep disorders are observed in 50-80% of children with ASD and are correlated with daytime problem behaviors. Furthermore, sleep problems exacerbate the severity of ASD core symptoms and are associated with behavioral disruptions in children with ASD (Massi et al. An Overview of Autism Spectrum Disorder, Heterogeneity and Treatment Options. Neurosci Bull. 2017 Apr; 33(2): 183-193). In addition, aberrant behaviors are also correlated with gastrointestinal problems in young children with ASD (Chaidez et al. Gastrointestinal problems in children with autism, developmental delays or typical development. J Autism Dev Disord. 2014; 44: 1117-1127), and markers of mitochondrial dysfunction are significantly correlated with autism severity (Rossignol et al. Mitochondrial dysfunction in autism spectrum disorders: a systematic review and meta-analysis. Mol Psychiatry. 2012; 17(3): 290-314).

The role of immune system abnormalities in ASD is a significant focus of ongoing research. Altered immunity involving cytokines, immunoglobulins, inflammation, cellular activation, and autoimmunity, have all been implicated in ASD (Goines, Van de Water. The immune system's role in the biology of autism. Curr Opin Neurol. 2010; 23: 111-117). Furthermore, altered levels of cytokines have been also associated with the severity of behavioral impairments (Ricci et al. Altered cytokine and BDNF levels in autism spectrum disorder. Neurotox Res. 2013 Nov;24(4):491-501; Jyonouchi et al. Cytokine profiles by peripheral blood monocytes are associated with changes in behavioral symptoms following immune insults in a subset of ASD subjects: an inflammatory subtype? J Neuroinflammation. 2014 Oct 27;11:187; Gladysz et al. Immune Abnormalities in Autism Spectrum Disorder-Could They Hold Promise for Causative Treatment? Mol Neurobiol. 2018 Aug;55(8):6387-6435).

There is currently no approved treatment to address the core symptoms of ASD. Antipsychotics have demonstrated efficacy on some of the associated behavioral problems, in particular irritability associated with ASD (Jobski et al. Use of psychotropic drugs in patients with autism spectrum disorders: a systematic review. Acta Psychiatr Scand, 2017. 135(1): p. 8-28). For instance, atypical neuroleptics and stimulants have been relatively effective for irritability/disruptive behavior and Attention-Deficit/Hyperactivity Disorder (ADHD) symptoms, respectively (Fung et al. Pharmacologic Treatment of Severe Irritability and Problem Behaviors in Autism: A Systematic Review and Meta-analysis. Pediatrics, 2016. 137 Suppl 2: p. S124-35). Among antipsychotics, risperidone and aripiprazole have been approved by the US Food and Drug Administration for the treatment of irritability in youth with ASD. Despite evidence of antipsychotic-associated efficacy in ASD, treatment response is highly variable and it is often associated with side effects, like sedation, risk of extrapyramidal symptoms, and metabolic abnormalities. In clinical studies of D-cycloserine and memantine, positive effects on core ASD symptoms were seen only in some adolescents and adults with ASD (Aman et al. Safety and Efficacy of Memantine in Children with Autism: Randomized, Placebo-Controlled Study and Open-Label Extension. J Child Adolesc Psychopharmacol. 2017 Jun;27(5):403-412; Schade et al.D-Cycloserine in Neuropsychiatric Diseases: A Systematic Review. Int J Neuropsychopharmacol. 2016 Apr 20;19(4)). In a study of a low-dose of sertraline in young boys with fragile X syndrome, an increase in social interaction was only seen in boys who had ASD and specific brain-derived neurotrophic factor polymorphisms (i.e., Met variants) (AlOlaby et al. Molecular biomarkers predictive of sertraline treatment response in young children with fragile X syndrome. Brain Dev. 2017 Jun;39(6):483-492).

In a first phase 2 double-blind placebo-controlled trial of arbaclofen, a potent and selective agonist of GABA receptors, overall results did not show any improvement on the primary outcome measured (Veenstra-VanderWeele et al. Arbaclofen in Children and Adolescents with Autism Spectrum Disorder: A Randomized, Controlled, Phase 2 Trial. Neuropsychopharmacology. 2017 Jun;42(7):1390-1398). However, a secondary analysis within the same study revealed significant improvements in CGI-I (clinical global impression of improvement scale) in a subgroup of patients. Although no biological hypothesises and/or evidences have been found yet to explain such heterogeneity, it appeared that the effect of arbaclofen was greater in higher functioning patients. Based on CGI-Severity, 13% of the participants showed a shift of two or more points within the arbaclofen group, suggesting a clinically meaningful change in global impairment in this subset of children. Positive responses in a small subset of individuals, but groupwise non statistically significant, are characteristic of most of these early pharmacological treatment trials in ASD.

Sulforaphane, a potent modulator of heat shock transcription factor 1 and heat-shock proteins, has been shown to improve behaviour in ASD patients (Singh et al. Sulforaphane treatment of autism spectrum disorder (ASD). PNAS 2014; 111:43, 15550-5). However, the authors likewise reported heterogenous responses, with either significant improvement, or no changes or even worsening of behavioural symptoms in participants.

More recently bumetanide, a fast-acting loop diuretic, has been evaluated in clinical trials as a potential treatment for autism. Lemonnier *et al.* conducted two randomized, double-blind, placebo-controlled trials evaluating the efficacy of bumetanide in children with autism (Lemonier et al. A randomised controlled trial of bumetanide in the treatment of autism in children. Transl Psychiatry. 2012; 11;2: e202; Lemonier et al. Effects of bumetanide on neurobehavioral function in children and adolescents with autism spectrum disorders. 2017; Translational Psychiatry volume 7, e1056). In both studies, after 3 months of treatment, bumetanide-treated groups showed clinically meaningful improvement in the Childhood Autism Rating Scale (CARS) total score (5-point improvement on average versus 2 points in placebo). In the second study, a deeper analysis revealed a proportion of "high CARS responders" at approx. 50% regardless of the dose group (versus 17% for the placebo group). Similarly, significant improvements on a more clinically meaningful scale, the clinical global impression of improvement (CGI-I), were observed in 52% of the bumetanide patients in the first study (versus 22% with placebo), and 33% (versus 5% with placebo) in the second study. After placebo correction, these CARS and CGI-I results suggest that approximately 30% of patients with ASD could respond to bumetanide. However, the authors were unable to identify which patients would respond to the treatment.

This heterogeneity of ASD patients with regards to the treatment response (illustrated by the arbaclofen or sulforaphane or bumetanide trials), further supports the urgent need to shift from the "one-size-fits-all" approach towards understanding molecular and genetic heterogeneity in order to provide the right treatment to the right patients.

As the underlying causes of ASD remain elusive, attempts have already been made to stratify ASD patients into smaller, more homogeneous subgroups, by focusing on specific genetic alteration signatures (Bernier et al., Disruptive CHD8 mutations define a subtype of autism early in development, Cell 2014 Jul 17; 158 (2): 263-276; Kuechler et al. Bainbridge-Ropers syndrome caused by loss-of-function variants in ASXL3: a recognizable condition. Eur J Hum Genet. 2017 Feb;25(2):183-191). Importantly, these analyses provide evidence for connections between specific genetic alterations in carriers of loss-of-function mutations in the ASXL3 gene and ASD. But beyond characterizing the connection between single genetic insults and observable phenotype outcomes, in order to develop drug treatments that are efficient for a specific and well-defined subset of ASD patients, it is key to also understand the underlying disrupted pathways in these patients. In this context, a method directed to identifying a subset of idiopathic ASD patients based on the clustering of clinical signs and symptoms which allows for the identification of dysregulated pathways and ultimately the identification of a specific treatment has been previously reported (EP18204763.9). However, given the heterogeneity of ASD, there is a need to identify further subtypes of ASD that will respond to specific treatments.

### Objective problem to be solved

The problem to be solved is thus the provision of means to efficiently identify a further subgroup of ASD patients and the provision of an effective treatment for this subgroup.

### Summary of invention

The invention is directed to a pharmaceutical composition comprising a substance capable of inducing repolarization of the membrane of excitable cells after depolarization for use in the treatment of autism spectrum disorder (ASD) phenotype 3.

Furthermore, the invention is directed to a pharmaceutical composition comprising a substance capable of inducing repolarization of the membrane of excitable cells after depolarization for use in the treatment of ASD in a patient, wherein the patient shows at least two of the following first group of clinical signs and symptoms comprising macrocephaly, tachycardia, long neck, hypophosphatemia, McCune-Albright syndrome, seizures, hypertension, hyperkalemia, gastroesophageal reflux, precocious puberty, hyperinsulinemia, increased inflammatory response, familial history of cholangiocarcinoma, hearing impairment, growth hormone excess, tall stature, hyperchloraemia and type II diabetes.

The invention is also directed to a method for dividing an ASD patient cohort into patients responding to treatment with a substance capable of inducing repolarization of the membrane of excitable cells after depolarization and patients not responding to said treatment, comprising the steps of:
- providing a patient database including data sets relating to the following first group of clinical signs and symptoms comprising macrocephaly, tachycardia, long neck, hypophosphatemia, McCune-Albright syndrome, seizures, hypertension, hyperkalemia, gastroesophageal reflux, precocious puberty, hyperinsulinemia, increased inflammatory response, familial history of cholangiocarcinoma, hearing impairment, growth hormone excess, tall stature, hyperchloraemia and type II diabetes; and
- classifying a patient as a patient responding to treatment with a substance capable of inducing repolarization of the membrane of excitable cells after depolarization if he shows at least two of the clinical signs and symptoms of said first group.

The invention is further directed to a method for diagnosing an ASD patient with ASD phenotype 3, comprising the steps of:
- providing a patient-specific data set relating to the following first group of clinical signs and symptoms comprising macrocephaly, tachycardia, long neck, hypophosphatemia, McCune-Albright syndrome, seizures, hypertension, hyperkalemia, gastroesophageal reflux, precocious puberty, hyperinsulinemia, increased inflammatory response, familial history of cholangiocarcinoma, hearing impairment, growth hormone excess, tall stature, hyperchloraemia and type II diabetes; and
- diagnosing the patient with ASD phenotype 3 if he shows at least two of the clinical signs and symptoms of said first group.

### Detailed description

The present invention relates to a pharmaceutical composition comprising a substance capable of inducing repolarization of the membrane of excitable cells after depolarization for use in the treatment of a specific subgroup of ASD patients, called ASD phenotype 3.

As used herein, the term autism spectrum disorder (ASD) is understood to cover a family of neurodevelopmental disorders characterized by deficits in social communication and interaction and restricted, repetitive patterns of behavior, interests or activities. In the following, the terms "autism spectrum disorder", "autism" and "ASD" are used interchangeably.

Herein, the terms "ASD Phenotype 3" and "Phenotype 3" are used interchangeably.

The term "patient" refers to "ASD patient" and is intended to cover not only humans diagnosed as having ASD, but also humans suspected of having ASD.

The person skilled in the art is well aware of how a patient may be diagnosed with ASD.

For example, the skilled person may follow the criteria set up in "American Psychiatric Association; Diagnostic and Statistical Manual of Mental Disorders (DSM-5) Fifth edition" to give a subject a diagnosis of ASD. Likewise, ASD patients may have been diagnosed according to standardized assessments tools including but not limited to CIM-10, ICD-10, DISCO, ADI-R, ADOS or CHAT.

In other cases, patients may have a well-established DSM-IV diagnosis of autistic disorder, Asperger's disorder, or pervasive developmental disorder not otherwise specified (PDD-NOS).

Additionally, the present invention may be useful for subjects fulfilling one or more of the following criteria: persistent deficits in social communication and social interaction across multiple contexts as manifested by the following, currently or by history; restricted, repetitive patterns of behavior, interests, or activities, as manifested by at least two of the following, currently or by history; symptoms present in the early development period (but may not become fully manifest until social demands exceed limited capacities, or maybe masked by learned strategies in later life); symptoms cause clinically significant impairment in social, occupational, or other important areas of current functioning; these disturbances are not better explained by intellectual disability (intellectual development disorder) or global development delay.

ASD may occur with or without accompanying intellectual and/or language impairment. It may be associated with a known medical or genetic condition or an environmental factor or other neurodevelopmental, mental or behavioral disorders.

ASD may occur in different severity levels which may be classified according to impairment in social communication and in terms of restricted, repetitive behavior. Importantly, the term ASD phenotype 1 is not associated with a particular severity level of ASD. The present invention may be applied to patients suffering from any severity level of ASD.

The present inventors have surprisingly identified a subgroup of ASD patients called ASD phenotype 3 which are characterized by a prolonged repolarization of the membrane of various cell types after depolarization (i.e. abnormally long lasting potentials) due to an altered expression of membrane channels or key regulators of membrane channels. So called channelopathy ultimately leads to altered release of neurotransmitters and hormones that trigger repeated membrane depolarization (Schmunk et al. Channelopathy pathogenesis in autism spectrum disorders. Front Genet. 2013 Nov 5;4:222). Such alterations are not restricted to neuronal cells, but also occur in other excitable cells such as beta pancreatic cells.

In ASD phenotype 3 patients, prolonged membrane depolarization causes abnormal brain development with pathological alterations in neuronal differentiation and maturation (Sohya et al. Chronic membrane depolarization-induced morphological alteration of developing neurons. Neuroscience. 2007 Mar 2;145(1):232-40), including a reduced number of cells expressing GABA and serotonin neurotransmitters (He at al. Prolonged membrane depolarization enhances midbrain dopamine neuron differentiation via epigenetic histone modifications. Stem Cells. 2011 Nov;29(11):1861-73). This therefore leads to excessive glutamate release, which promotes inflammation (Wen et al. Excitatory amino acid glutamate: role in peripheral nociceptive transduction and inflammation in experimental and clinical osteoarthritis. Osteoarthritis Cartilage. 2015 Nov;23(11):2009-16; Osikowicz et al. The glutamatergic system as a target for neuropathic pain relief. Exp Physiol. 2013 Feb;98(2):372-84; Swaim et al. Platelets contribute to allograft rejection through glutamate receptor signaling. J Immunol. 2010 Dec 1;185(11):6999-7006) and triggers repeated membrane depolarization. In ASD phenotype 3 patients, prolonged membrane depolarization also causes hyperinsulinemia (Daraio et al. SNAP-25b-deficiency increases insulin secretion and changes spatiotemporal profile of Ca2+oscillations in β cell networks. Sci Rep. 2017 Aug 10;7(1):7744). Chronic (or congenital) hyperinsulinemia leads to recurrent episodes of hypoglycemia, which decreases the capacity of energy production in the brain; this can in turn lead to failure of the ATP-dependent sodium-potassium pumps, and therefore exacerbate the excess of membrane depolarization by making the cells incapable of maintaining a stable resting membrane potential (Abend et al. Chapter 12 - Neonatal Seizures. Volpe's Neurology of the Newborn (Sixth Edition) 2018, Pages 275-321.e14), thus increasing the risk for seizures (Sousa-Santos et al. Congenital hyperinsulinism in two siblings with ABCC8 mutation: same genotype, different phenotypes. Arch Endocrinol Metab. 2018 Oct;62(5):560-565; Aka et al. Seizures and diagnostic difficulties in hyperinsulinism-hyperammonemia syndrome. Turk J Pediatr. 2016;58(5):541-544). In the long term, hyperinsulinemia can lead to insulin resistance and type 2 diabetes, resulting in recurrent episodes of hyperglycemia (Shannik et al. Insulin resistance and hyperinsulinemia: is hyperinsulinemia the cart or the horse? Diabetes Care. 2008 Feb;31 Suppl 2:S262-8).

It is therefore an aim of the invention to provide these patients with a novel treatment directed to mitigating the effects of prolonged membrane depolarization. This is achieved by administration of a substance capable of inducing repolarization of the cell membrane after depolarization.

Herein the term "substance capable of inducing repolarization of the cell membrane after depolarization" is understood to relate to substances that help restore the cell resting membrane potential to its physiological range by counteracting abnormally prolonged repolarizations (i.e. abnormally long lasting action potentials). Prolonged repolarization is often caused by a channelopathy leading to an abnormal release of neurotransmitters and hormones and an excess of membrane depolarization. The person skilled in the art is well aware of how the altered ion homeostasis, membrane channel expression and receptor-operated channel can modify cell membrane potential and cause dysfunction of several cellular activities such as gene expression, mitochondrial function, protein synthesis, protein trafficking, or neurotransmitter release.

In one aspect of the invention, the excitable cells are neuronal and beta pancreatic cells, i.e. the pharmaceutical compositions comprises a substance capable of inducing repolarization of the membrane of neuronal and beta pancreatic cells.

Since the mechanisms described above apply in particular to the the subgroup of ASD phenotype 3 patients, the invention is directed to pharmaceutical compositions comprising substances capable of inducing repolarization of the membrane of excitable cells for use in ASD phenotype 3 patients.

According to the invention, ASD phenotype 3 patients can be identified by checking for the presence of one or more clinical signs and symptoms of a first group comprising macrocephaly, tachycardia, long neck, hypophosphatemia, McCune-Albright syndrome, seizures, hypertension, hyperkalemia, gastroesophageal reflux, precocious puberty, hyperinsulinemia, increased inflammatory response, familial history of cholangiocarcinoma, hearing impairment, growth hormone excess, tall stature, hyperchloraemia and type II diabetes.

Herein, it is defined that a patient shows the clinical sign and symptom "macrocephaly" if the circumference of the patient's head is more than two standard deviations above average for their age and gender or if their head is larger than the 98th percentile.

Herein, it is defined that a patient shows the clinical sign and symptom "long neck" if the patient's neck measures longer than the width of 4 extended fingers of his/her palm (without considering the thumb).

Herein, it is defined that a patient shows the clinical sign and symptom "precocious puberty" if the onset of puberty happens before the age of 8 years in girls or 9 years in boys.

Herein, it is defined that a patient shows the clinical sign and symptom "hyperinsulinemia" if the patient has an elevated concentration of insulin in the blood compared to the normal/expected range as defined in Table 1.

**Table 1.- Reference ranges of insulin and glucose levels for children over the age of 2 years to adult (Pagana et al. Mosby's Diagnostic & Laboratory Test Reference. 14th ed. St. Louis, Mo: Elsevier; 2019; Yorifuji. Congenital hyperinsulinism: current status and future perspectives. Ann Pediatr Endocrinol Metab. 2014 Jun; 19(2): 57-68.)**

| | Insulin level | Glucose level |
|---|---|---|
| Hypoglycemia | < 20.84 pmol/L | < 2.5 mmol/L |
| Fasting (ie., no caloric intake for at least 8 hours) | < 174 pmol/L | < 6.1 mmol/L |
| 2 hours after glucose administration | 111-1153 pmol/L | < 7.8 mmol/L |

Herein, it is defined that a patient shows the clinical sign and symptom "increased inflammatory response" if the patient shows an overproduction of immune cells or proteins including Th1 cytokines, a family history of autoimmune disorders, or worsening of ASD symptoms under sickness.

Herein, it is defined that a patient shows the clinical sign and symptom "hearing impairment" if he/she shows a decreased magnitude of the sensory perception of sound.

Herein, it is defined that a patient shows the clinical sign and symptom "tall stature" if the patient's height is more than two standard deviations above the mean according to age and gender norms.

Herein, it is defined that a patient shows the clinical sign and symptom "tachycardia" if the patient's heart rate exceeds the normal resting state according to age as defined in Table 2.

**Table 2. Normal pulse rates at rest, in beats per minute (BPM; U.S. Department of Health and Human Services - National Ites of Health)**

| newborn (0-3 months old) | infants (3 - 6 months) | infants (6 - 12 months) | children (1 - 10 years) | children over 10 years & adults, including seniors | well-trained adult athletes |
|---|---|---|---|---|---|
| 99-149 | 89-119 | 79-119 | 69-129 | 59-99 | 39-59 |

Herein, it is defined that a patient shows the clinical sign and symptom "seizures" if the patient has ever expirienced sudden attacks with physical manifestations such as convulsions, sensory disturbances, or loss of consciousness, resulting from abnormal electrical discharges in the brain.

Herein, it is defined that a patient shows the clinical sign and symptom "hypertension" if the patient has as a systolic blood pressure (SBP) of 140 mm Hg or more, or a diastolic blood pressure (DBP) of 90 mm Hg or more (for adults; Chobanian et al. Seventh report of the Joint National Committee on Prevention, Detection, Evaluation, and Treatment of High Blood Pressure. Hypertension. 2003 Dec. 42(6):1206-52), or if the patient's blood pressure is above the 95^{th} percentile according to age (for children; Flynn et al. Clinical Practice Guideline for Screening and Management of High Blood Pressure in Children and Adolescents. Pediatrics September 2017, 140 (3) e20171904).

Herein, it is defined that a patient shows the clinical sign and symptom "hypophosphatemia" if the patient has a serum phosphate level of less than 2.5 mg/dL (adult) or less than 4 mg/dL (children; Sharma et al. Hypophosphatemia. St Treasure Island (FL): StatPearls Publishing).

Herein, it is defined that a patient shows the clinical sign and symptom "McCune-Albright syndrome" if the patient has ever received such medical diagnosis or shows symptoms fitting the diagnosis of "McCune-Albright syndrome".

Herein, it is defined that a patient shows the clinical sign and symptom "hyperkalemia" if the patient has a serum potassium level above 5.0 mEq/L (for children over the age of 2 years to adults; Simon et al. Hyperkalemia. St Treasure Island (FL): StatPearls Publishing)

Herein, it is defined that a patient shows the clinical sign and symptom "gastroesophageal reflux" if the patient suffers from a burning feeling in his/her chest or throat due to acidic stomach juices leaking backwards from the stomach into the esophagus through the lower esophageal sphincter.

Herein, it is defined that a patient shows the clinical sign and symptom "growth hormone excess" if the patient has a serum growth hormone level > 226 pmol/L (in adult men), > 452 pmol/L (in adult women), or > 904 pmol/L (in children).

Herein, it is defined that a patient shows the clinical sign and symptom "hyperchloraemia" if the patient has a serum chloride concentration above 106 mEq/L (Morrison. Clinical Methods: The History, Physical, and Laboratory Examinations. 3rd edition. Chapter 197. Boston: Butterworths).

Herein, it is defined that a patient shows the clinical sign and symptom "type II diabetes" if the patient has ever received such medical diagnosis or shows symptoms fitting the diagnosis of "type II diabetes".

In a preferred embodiment, an ASD phenotype 3 patients shows at least two, three or four of clinical signs and symptoms of the first group. In a particularly preferred embodiment, an ASD phenotype 3 patients shows hyperinsulinemia and at least one other clinical sign and symptom of the first group. This allows for a particularly reliable and easy identification of ASD phenotype 3 patients.

In another preferred embodiment, an ASD phenotype 3 patient does not show any of clinical signs and symptoms of the second group comprising type I diabetes, obesity, progressive ossesous heteroplasia, Albright hereditary osteodystrophy, osteoma cutis, microcephaly, nystagmus, hypokalemia, hypotension, immunodeficiency, dyskinesia, brachydactyly, short neck, hyperphosphatemia, growth hormone deficiency, short stature and hypochloraemia.

Herein, it is defined that a patient shows the clinical sign and symptom "microcephaly" if the circumference of the patient's head is more than two standard deviations below average for the age and gender or if their head is smaller than the 2th percentile.

Herein, it is defined that a patient shows the clinical sign and symptom "short neck" if the patient's neck measures less than the width of 4 extended fingers of his/her palm (without considering the thumb).

Herein, it is defined that a patient shows the clinical sign and symptom "immunodeficiency" if the patient has an underproduction of immune-system cells and/or proteins including T-lymphocytes (Valiathan et al. Reference ranges of lymphocyte subsets in healthy adults and adolescents with special mention of T cell maturation subsets in adults of South Florida. Immunobiology. 2014 Jul;219(7):487-96), recurrent and/or long lasting infections, or improvement of ASD symptoms under sickness.

Herein, it is defined that a patient shows the clinical sign and symptom "short stature" if the patient's height is more than two standard deviations below the mean according to age and gender norms.

Herein, it is defined that a patient shows the clinical sign and symptom "type I diabetes" if if the patient has ever received such medical diagnosis or shows symptoms fitting the diagnosis of "type I diabetes".

Herein, it is defined that a patient shows the clinical sign and symptom "obesity" if the patient has a BMI above 30 kg/m2 (World Health Organization).

Herein, it is defined that a patient shows the clinical sign and symptom "progressive ossesous heteroplasia" if if the patient has ever received such medical diagnosis or shows symptoms fitting the diagnosis of "progressive ossesous heteroplasia".

Herein, it is defined that a patient shows the clinical sign and symptom "Albright hereditary osteodystrophy" if if the patient has ever received such medical diagnosis or shows symptoms fitting the diagnosis of "Albright hereditary osteodystrophy".

Herein, it is defined that a patient shows the clinical sign and symptom "osteoma cutis" if the patient has ever received such medical diagnosis or shows symptoms fitting the diagnosis of "osteoma cutis".

Herein, it is defined that a patient shows the clinical sign and symptom "nystagmus" if the patient has rhythmic, involuntary oscillations of one or both eyes related to abnormality in fixation, conjugate gaze, or vestibular mechanisms.

Herein, it is defined that a patient shows the clinical sign and symptom "hypokalemia" if the patient has a serum potassium level below 3.5 mEq/L (for children over the age of 2 years to adults; Simon et al. Hyperkalemia. St Treasure Island (FL): StatPearls Publishing).

Herein, it is defined that a patient shows the clinical sign and symptom "hypotension" if if the patient has as a systolic blood pressure (SBP) below 90 mm Hg, or a diastolic blood pressure (DBP) below 60 mm (for children over the age of 10 years to adult).

Herein, it is defined that a patient shows the clinical sign and symptom "dyskinesia" if the patient has diminished voluntary movements and the presence of involuntary movements.

Herein, it is defined that a patient shows the clinical sign and symptom "brachydactyly" if the patient has digits that appear disproportionately short compared to the hand/foot.

Herein, it is defined that a patient shows the clinical sign and symptom "hyperphosphatemia" if patient has a serum phosphate level above 4.5 mg/dL (adult) or above 7 mg/dL (children; Sharma et al. Hypophosphatemia. St Treasure Island (FL): StatPearls Publishing).

Herein, it is defined that a patient shows the clinical sign and symptom "growth hormone deficiency" if the patient has a serum growth hormone level below 0.32 pmol/L (Darendeliler et al. Reevaluation of growth hormone deficiency during and after growth hormone (GH) treatment: diagnostic value of GH tests and IGF-I and IGFBP-3 measurements. J Pediatr Endocrinol Metab. 2004 Jul;17(7):1 007-12).

Herein, it is defined that a patient shows the clinical sign and symptom "hypochloraemia" if the patient has a serum chloride concentration below 90 mEq/L.

The combination of positive and negative clinical signs and symptoms allows for an even better differentiation between ASD phenotype 3 patients and other idiopathic ASD patients.

According to the invention, ASD phenotype 3 patients can be treated by and respond positively to administration of substances capable of stabilizing the resting membrane potential of excitable cells such as neuronal and pancreatic cells.

The person skilled in the art is aware how a substance is capable of inducing repolarization of the membrane of excitable cells after depolarization. For example, the substance may contribute to repolarization by balancing the ion concentration gradient by modulating voltage-gated cation channels activity through the transcriptional up-regulation of genes such as SNAP25, KCNJ6, KCNQ3, KCNK3, KCNJ10, and KCNV1. Alternatively, channel activity can also be modulated by down-regulation of GNAS. Likewise, substances decreasing the release of agonists, such as insulin, may also contribute to repolarization by the transcriptional up-regulation of SNAP25 and SLC12A5 (KCC2), and down-regulation of GNAS.

The person in the art therefore also knows how substances capable of contributing to repolarization the membrane of excitable cells after depolarization can be identified. For example, the person skilled in the art can identify such substances by testing whether the substance upregulates SNAP25, KCNJ6, KCNQ3, KCNK3, KCNJ10, and/or KCNV1 or downregulates GNAS and/or MIF. Such an analysis is within the routine measures of the skilled person.

Substances capable of inducing repolarization of the membrane of excitable cells after depolarization include loop diuretics and thiazide diuretics and may be selected from the groups of sulfanilamides, in particular azosemide, benzmetanide, clofenamide, clopamide, clorexolone, furosemide, indapamide, mefruside, metolazone, piretanide, torasemide (torsemide) and xipamide; meta-aminobenzoates, in particular bumetanide; benzothiadiazines, in particular bendroflumethiazide, chlorothiazide, cyclopenthiazide, cyclothiazide, hydrochlorothiazide, hydroflumethiazide, mebutizide, methylclothiazide, polythiazide and trichlormethiazide; thiazoles, in particular etozolin, ozolinone and tizolemide; quinazolines, in particular fenquizone and quinethazone; phthalimides, in particular chlortalidone; phenoxyacetates, in particular etacrynic acid; indans, in particular indocrinone; pyrazoles, in particular muzomimine; indoles, in particular tripamide.

In a preferred embodiment of the invention, the substance capable of inducing repolarization of the membrane of excitable cells after depolarization is bumetanide, AqB007, AqB011, PF-2178, BUM13, BUM5, bumepamine or a mixture thereof. In a particularly preferred embodiment, the substance capable of inducing repolarization of the membrane of excitable cells is bumetanide, 3-(Butylamino)-4-phenoxy-5-sulfamoylbenzoic acid, a potent loop diuretic.

The pharmaceutical composition according to the invention is to be administered in a therapeutically effective amount. For example, 0.01 to 10 mg of the pharmaceutical composition according to the invention may be administered in a single daily dose. In preferred embodiments, the pharmaceutical composition according to the invention is administered as a total daily dose of 0.5 to 10 mg, more preferably 0.5 to 6 mg, most preferably 0.5. to 4 mg either once or twice daily.

The person skilled in the art is aware of ways and methods to administer the pharmaceutical composition according to the invention. For example, the pharmaceutical composition according to the invention is administered orally, nasally, parenterally or intravenously. In a preferred embodiment, the pharmaceutical composition according to the invention is administered orally.

In order to achieve the desired dosage level, the pharmaceutical composition according to the invention may be administered once daily or in several doses per day.

In a preferred embodiment, the pharmaceutical composition according to the invention comprises bumetanide and is admininistered 0.5 to 2 mg orally once daily or 0.5. to 1 mg intravenously or intramuscularly. In one embodiment, the pharmaceutical composition is administered as a continuous intravenous infusion with 1 mg/hour up to 12 mg/day.

In another embodiment, the pharmaceutical composition is for use in the treatment of pediatric patients younger than 6 months, wherein the treatment comprises administration of 0.01 to 0.05 mg/kg, preferably 0.04 mg/kg bumetanide, once daily or every other day. In another embodiment the pharmaceutical composition is for use in the treatment of pediatric patients older than 6 months, wherein the treatment comprises administration of 0.015 to 0.1 mg/kg bumetanide once daily or every other day.

The pharmaceutical composition according to the invention may additionally comprise pharmacologically acceptable excipients such as fillers, disintegrants, lubricants, adhesives, anti-adherents, binders, coatings, glidants, sweeteners, flavors, sorbents, colors and preservatives. The person skilled in the art knows how to formulate an active ingredient in a way to facilitate drug absorption, reduce viscosity or enhance solubility.

In one embodiment, the pharmaceutical composition comprising a substance capable of stabilizing the resting membrane potential of neuronal and pancreatic cells is used in the treatment of ASD in a patient, wherein the treatment comprises
- determining whether the patient suffers from ASD phenotype 3 and
- administering a therapeutically effective amount of the pharmaceutical composition to the patient if the patient suffers from ASD phenotype 3,
wherein determining whether the patient suffers from ASD phenotype 3 comprises checking for upregulation of GNAS and/or MIF, and/or checking for the downregulation of CTNNA2, GABRA1, NR3C1, SLC12A5, KCNJ6, KCNQ3, KCNJ10, DRD1 and/or SNAP25. Herein, GNAS refers to the heterotrimeric G-protein alpha subunit, MIF refers to macrophage migration inhinitory factor, CTNNA2 refers to alpha-2-catenin, GABRA1 refers to gamma-aminobutyric acid receptor subunit alpha-1, NR3C1 refers to glucocorticoid receptor (nuclear receptor subfamily 3, group C, member 1), SLC12A5 refers to chloride potassium transporter member 5, KCNJ6 refers to G protein-activated inward rectifier potassium channel 2, KCNQ3 refers to Kv7.3 potassium channel, KCNJ10 refers to ATP-sensitive inward rectifier potassium channel 10, DRD1 refers to dopamine receptor D1, and SNAP25 refers to synaptosomal nerve-associated protein 25.

The skilled person is well aware how an analysis of upregulation and downregulation of specific genes can be performed. For example, it may be checked on the mRNA level using quantitative PCR techniques such as qPCR or RT-qPCR or semi-quantitative techniques such as RNA sequencing or Fluorescence In Situ Hybridization (FISH) techniques. Likewise, up- or downregulation of a gene may be determined on the protein level using protein quantification techniques such as Western Blot and quantitative dot blots. Upregulation is understood to mean an increase of mRNA levels or protein levels of at least 20% when compared to controls. Downregulation is understood to mean a decrease of mRNA levels or protein levels of at least 20% when compared to controls.

In another embodiment, it is determined that the patient suffers from ASD phenotype 3 if he shows at least two of the clinical signs and symptoms of the first group, namely macrocephaly, tachycardia, long neck, hypophosphatemia, McCune-Albright syndrome, seizures, hypertension, hyperkalemia, gastroesophageal reflux, precocious puberty, hyperinsulinemia, increased inflammatory response, familial history of cholangiocarcinoma, hearing impairment, growth hormone excess, tall stature, hyperchloraemia and type II diabetes.

The present invention is also directed to a method for dividing an ASD patient cohort into patients responding to treatment with a substance capable of inducing repolarization of the membrane of excitable cells after depolarization and patients not responding to said treatment, comprising the steps of:
- providing a patient database including data sets relating to the following first group of clinical signs and symptoms comprising macrocephaly, tachycardia, long neck, hypophosphatemia, McCune-Albright syndrome, seizures, hypertension, hyperkalemia, gastroesophageal reflux, precocious puberty, hyperinsulinemia, increased inflammatory response, familial history of cholangiocarcinoma, hearing impairment, growth hormone excess, tall stature, hyperchloraemia and type II diabetes ; and
- classifying a patient as a patient responding to treatment with a substance capable of inducing repolarization of the membrane of excitable cells after depolarization, if he shows at least two of the clinical signs and symptoms of said first group.

Herein, the term "respond" is understood to mean that a patient's conditions improves upon administration of the treatment, whereas the term "non-responding" is meant to cover patients showing no improvement or even worsening of the condition upon administration. In particular, the term "respond" is defined as showing an improvement of ASD core and ancillary symptoms upon administration of the pharmaceutical composition according to the invention. ASD core symptoms include but are not limited to social-interaction difficulties, communication challenges and a tendency to engage in repetitive behavior. ASD ancillary symptoms include but are not limited to intellectual disability, learning disability, genitourinary organ dysfunction (i.e. impairment to initiate urinating), episodes of diarrhea.

The present invention is therefore useful for stratifying a general population of ASD patients into a subgroup that responds to treatment with a substance capable of inducing repolarization of the membrane of excitable cells after depolarization and a subgroup that does not respond to said treatment. Such a stratification is advantageous when designing clinical studies because given the heterogeneous nature of ASD, many treatments will show efficacy only in a subgroup of patients. Therefore, if a non-stratified patient population is used for a clinical study, treatment efficacy in a subgroup of patients can be masked by non-efficacy in the rest of the patient population.

A response may also comprise a decrease in non-compliance/disruptive behaviors, repetitive behaviors and restricted interests (as measured by the ADI-R, ABC (Aberrant Behavior Checklist)- ABC specific subscales (i.e. ABC-I) as well as worsening of preexisting qualitative and quantitative communication and social deficits as measured by Social Responsiveness Scale (SRS).

Particularly, responders to a treatment may display an increase in social responsiveness characterized by but not limited to decreased latency to respond or establish eye contact and/or improved initiation of speech measured by mean number of verbal initiation within a given time interval in a similar contextual environment, decreased latency to response to verbal initiation, increase in executive functioning including increased ability to plan and implement multiple step tasks, increase in behavioral compliance, decreased irritability (i.e. ABC irritability subscale), lowered sensitivity to sensory stimuli, increase in short term memory retention and marked decrease or improvement in intensity of idiosyncratic behaviors and postures.

A positive response may also comprise or consist of positive variation in scores in standardized tests such as:
Autism Diagnostic Interview-Revised (ADI-R): is a standardized, semi-structured clinician led parent interview. The ADI-R includes 93 items focusing on Early Development, Language/Communication, Reciprocal Social Interactions, and Restricted, Repetitive Behaviors and Interests. (ADI-R; Rutter et al. 2003). It is divided into four different modules.

Aberrant Behavior Checklist (ABC): is a symptoms rating checklist used to assess and classify problem behaviors of children and adults in a variety of settings. The ABC includes 58 items that resolve into five subscales: (1) irritability, (2) lethargy/social withdrawal, (3) stereotypic behavior, (4) hyperactivity/noncompliance, and (5) inappropriate speech. A negative change signifies improvement and a positive change signifies worsening. The value for determining positive or negative response is the change from baseline to 3-5 days after administration of the Nrf2-activator according to the present invention.

Social Responsiveness Scale (SRS). The 65-item SRS is a standardized measure of the core symptoms of autism. Each item is scored on a 4-point Likert scale. The score of each individual item is summed to create a total raw score. Total scores results are as follows:
- 0-62: Within normal limits
- 63-79: Mild range of impairment
- 80-108: Moderate range of impairment
- 109-149: Severe range of impairment

The value for determining positive or negative response is the change from baseline to 3-5 days after treatment according to the present invention. The total score ranges from 0-149. A negative change signifies improvement and a positive change signifies worsening.

Clinical Global Impression Severity Scale (CGI-S): The CGI-S is a 7-point scale that requires the clinician to rate the severity of the patient's illness at the time of assessment, relative to the clinician's past experience with patients who have the same diagnosis. Considering total clinical experience, a patient is assessed on severity of mental illness at the time of rating:
- 1: normal, not at all ill
- 2: borderline mentally ill
- 3: mildly ill
- 4: moderately ill
- 5: markedly ill
- 6: severely ill
- 7: extremely ill

The value for determining positive or negative response is the change from baseline to 3-5 days after treatment according to the present invention. The total score ranges from 1-7. A negative change signifies improvement and a positive change signifies worsening.

Clinical Global Impression Improvement Scale (CGI-I): The CGI-I is a 7-point scale measure of overall change of Parent Target Problems (the child's two most pressing problems at screening, as reported by parents), using scores from the Clinical Global Impressions - Severity scale (CGI-S). Scores range from:
- 1: very much improved
- 2: much improved
- 3: minimally improved
- 4: no change
- 5: minimally worse
- 6: much worse
- 7: very much worse

The value for determining positive or negative response is the change from baseline to 3-5 days after treatment according to the present invention. The total score ranges from 1-7. A negative change signifies improvement and a positive change signifies worsening.

Autism Diagnostic Observation Schedule (ADOS): The ADOS is a semi-structured standardized assessment of communication and social interaction that is considered a gold-standard assessment of ASD. It is administered to diagnose autism and determine the level of severity of ASD in patients by evaluating impairments in two specific domains (the Social affect domain and the Restricted and Repetitive 25 behaviors domain). Different modules are used according to age and characteristics of the patients:
- Module 1: children who use little or no phrase speech
- Module 2: subjects who use phrase speech but do not speak fluently
- Module 3: younger subjects who are verbally fluent
- Module 4: adolescent and adults who are verbally fluent
and involve expert clinical judgement of the qualities and behaviors that are at the core of the social/communicative deficits. A negative change signifies improvement and a positive change signifies worsening. The ADOS scoring algorithm consists in 2 raw domain scores that are calibrated on a single 10-point scale, the ADOS-CSS (Calibrated Severity Scores).

Individuals with Scores between 6-10 on ADOS-CSS receive the classification "Autism", those with a CSS of 4-5 "ASD" down to "Nonspectrum" for individuals with CSS of 1-3. The value for determining positive or negative response is the change from baseline to 3-5 days after administration of the Nrf2-activator according to the present invention.

Childhood Autism rating Scales (CARS), (Schopler et al. Toward objective classification of childhood autism: Childhood Autism Rating Scale (CARS). J Autism Dev Disord. 1980 Mar;10(1):91-103). The CARS consists of 14 domains assessing behaviors associated with autism, with a 15th domain rating general impressions of autism. Each domain is scored on a scale ranging from one to four; higher scores are associated with a higher level of impairment. Total scores can range from a low of 15 to a high of 60; scores below 30 indicate that the individual is in the non-autistic range, scores between 30 and 36.5 indicate mild to moderate autism, and scores from 37 to 60 indicate severe autism (Schopler et al. The Childhood autism rating sclares: (CARS). Loas Angeles, Calif.: Western Psychological Services, 1988).

Other means to measure a change in ASD symptoms include: mean length of utterance (MLU) defined as the number of words or morphemes in each of spontaneous utterances. This measure is one of the most robust indices of young children's language acquisition. MLU is used to diagnose language impairments in young children, often defined as an MLU level one standard deviation or more below the mean for the child's age level. Additionally, intellectual functioning as a core symptom of ASD may be measured via the Vineland Adaptive Behavior Scale II (VABS-II), wherein a raise in scores signifies improvement, i.e. positive response.

A positive response may also comprise a positive change in one or more indicators measuring core or ancillary symptoms of ASD including: changes in observer reported or computer monitored eye tracking revealing level of interest in social stimuli versus objects, engagement in and responsiveness to social interaction, verbal and nonverbal communication abilities, occurrence of repetitive behaviors, level of intellectual functioning, measurement of attention, quality of motor coordination, quality of sleep, and intensity of symptoms of medical comorbidities (e.g. gastro-intestinal symptoms).

For example, a positive response as defined herein may be identified by a decrease of at least 10%, 20%, 30%, 40%, 50%, 60%, 70%, 80%, or 90% in any of ADI-R ABC, ABC-I, SRS, CARS or ADOS scores. In a preferred embodiment, the positive response comprises an decrease of at least 10% in total scores in these scales or any increase corresponding to a "minimally improved" (3) and preferably "much improved" (2) or "very much improved" (1) rating on the CGI-I scale.

In another embodiment, the present invention is directed to a method for diagnosing a patient with ASD phenotype 3, comprising the steps of:
- providing a patient-specific data set relating to the following first group of clinical signs and symptoms comprising macrocephaly, tachycardia, long neck, hypophosphatemia, McCune-Albright syndrome, seizures, hypertension, hyperkalemia, gastroesophageal reflux, precocious puberty, hyperinsulinemia, increased inflammatory response, familial history of cholangiocarcinoma, hearing impairment, growth hormone excess, tall stature, hyperchloraemia and type II diabetes; and
- diagnosing the patient with ASD phenotype 3 if he shows at least two of the clinical signs and symptoms of said first group.

In a preferred embodiment, a patient is diagnosed or classified as ASD phenotype 3 if he shows hyperinsulinemia and at least one other of the clinical signs and symptoms of said first group. In another embodiment, a patient is diagnosed or classified as ASD phenotype 3 if he shows at least three of the clinical signs and symptoms of said first group.

### Example

### Material and Methods

To characterize cell line invariant mechanism of action, bumetanide was tested across different human cell lines spanning several cancer cell lines (such as A549, MCF7, PC3, and VAP) as well as cells from healthy tissues (such as ASC, HA1E, and HT29). These cell lines (Table 3) were chosen due to their ease of manipulation and suitability for systematic screening. The growth medium used for the cell lines varies according to the different cell type characteristics, as suggested by the cell purchaser company recommendations. Incubation of cell types was performed inside an incubator with a humified atmosphere of 95% and 5% of CO2, and a constant temperature of 37°C. Cell maintenance medium can vary according to the nature of the different cell types.

Drug cell line treatments were performed using a dilution of 10 uM for all replicates at a time duration of 6 or 24 hours. Profiling drugs across a variety of these cell lines and treatment conditions (such as dose and time) provides a representative biological context for characterizing the cell line invariant mechanism of action as demonstrated previously (lorio et. al. Discovery of drug mode of action and drug repositioning from transcriptional responses, Proc Natl Acad Sci USA. 107(33):14621-6, 2010 Aug 17; lorio et. al. Transcriptional data: a new gateway to drug repositioning? Drug Discovery Today, 18, 7-8, 2013; Carrella D et al. Mantra 2.0: an online collaborative resource for drug mode of action and repurposing by network analysis, Bioinformatics 30 (12): 1787-1788, 2014; Sirci et. al. Computational Drug Networks: a computational approach to elucidate drug mode of action and to facilitate drug repositioning for neurodegenerative diseases, Drug Discovery Today: Disease Model, 19, 11-17, 2016; Pushpakom et. al. Drug repurposing: progress, challenges and recommendations, Nature reviews Drug discovery 18, 1, 41-58, 2019).

**Table 3. Time duration and dosage used for treating cell lines with bumetanide**

| **Cell line** | **Cell type** | **Reference** | **Time duration** | **Dosage** |
|---|---|---|---|---|
| A569 | Non-small cell lung carcinoma | ATCC CCL-185 | 6 hours | 10 uM |
| ASC | Adipocyte | From Jeff Gimble MD PhD, PBRC | 24 hours | 10 uM |
| HA1E | Kidney | CVCL_VU89 | 6 hours | 10 uM |
| HT29 | Intestine | ATCC HTB-38 | 6 hours | 10 uM |
| MCF7 | Breast adenocarcioma | ATCC HTB-22 | 6 hours | 10 uM |
| PC3 | Prostate adenocarcinoma | ATCC CRL-1435 | 24 hours | 10 uM |
| VCAP | Prostate carcinoma | ATCC CRL-2876 | 6 hours | 10 uM |

### Results

The ranking of genes for which up- or down-regulation of expression is associated with the capacity to repolarize the membrane of excitable cells after depolarization is listed in Table 4.

**Table 4. Ranking of the differential expressed genes (DEG) after bumetanide treatment in cell lines.**

| **Gene** | **DEG ranking after treatment** | **Direction of differential expression** |
|---|---|---|
| SNAP25 | 1 | UP |
| KCNJ6 | 19 | UP |
| SLC12A5 | 35 | UP |
| (KCC2) | | |
| KCNQ3 | 48 | UP |
| KCNK3 | 49 | UP |
| KCNJ10 | 113 | UP |
| KCNV1 | 165 | UP |
| GNAS | 1 | DOWN |

These results provide evidence for bumetanide having the capacity to contribute to repolarize excitable cells after depolarization mainly by:
1. up-regulating genes that encode for potassium voltage-gated channels whose main function is to contribute to repolarize excitable cells after depolarization, including KCNQ3, KCNK3, and KCNV1, as well as inward-rectifier potassium channels that help to restore the cells resting membrane potential, including KCNJ6 and KCNJ10;
2. regulating genes that have the capacity to decrease calcium influx and therefore shorten the duration of action potentials and decrease the release of neurotransmitters and hormones that can further trigger depolarization of excitable cells (Mansvelder et al. The relation of exocytosis and rapid endocytosis to calcium entry evoked by short repetitive depolarizing pulses in rat melanotropic cells. J Neurosci. 1998 Jan 1;18(1):81-92; Mansvelder et al. All classes of calcium channel couple with equal efficiency to exocytosis in rat melanotropes, inducing linear stimulus-secretion coupling. J Physiol. 2000 Jul 15;526 Pt 2:327-39), through the transcriptional up-regulation of genes such as SNAP25 and SLC12A5, and the transcriptional down-regulation of GNAS.

## Claims

1. Pharmaceutical composition comprising a substance capable of inducing repolarization of the membrane of excitable cells after depolarization for use in the treatment of autism spectrum disorder (ASD) phenotype 3.

2. Pharmaceutical composition comprising a substance capable of inducing repolarization of membrane of excitable cells after depolarization for use in the treatment of ASD in a patient, wherein the patient shows at least two of the following first group of clinical signs and symptoms comprising macrocephaly, tachycardia, long neck, hypophosphatemia, McCune-Albright syndrome, seizures, hypertension, hyperkalemia, gastroesophageal reflux, precocious puberty, hyperinsulinemia, increased inflammatory response, familial history of cholangiocarcinoma, hearing impairment, growth hormone excess, tall stature, hyperchloraemia and type II diabetes.

3. Pharmaceutical composition according to claim 2, wherein the patient does not show any of the following second group of clinical signs and symptoms comprising type I diabetes, obesity, progressive ossesous heteroplasia, Albright hereditary osteodystrophy, osteoma cutis, microcephaly, nystagmus, hypokalemia, hypotension, immunodeficiency, dyskinesia, brachydactyly, short neck, hyperphosphatemia, growth hormone deficiency, short stature and hypochloraemia.

4. Pharmaceutical composition comprising a substance capable of inducing repolarization of membrane of excitable cells after depolarization for use in the treatment of ASD in a patient, wherein the treatment comprises
- determining whether the patient suffers from ASD phenotype 3 and
- administering a therapeutically effective amount of the pharmaceutical composition to the patient if the patient suffers from ASD phenotype 3,
wherein determining whether the patient suffers from ASD phenotype 3 comprises checking for the upregulation of GNAS and/or MIF, and/or checking for the downregulation of CTNNA2, GABRA1, NR3C1, SLC12A5, KCNJ6, KCNQ3, KCNK3, KCNJ10, KCNV1, DRD1 and/or SNAP25.

5. Pharmaceutical composition comprising a substance capable of inducing repolarization of membrane of excitable cells after depolarization for use in the treatment of ASD in a patient, wherein the treatment comprises
- determining whether the patient suffers from ASD phenotype 3 and
- administering a therapeutically effective amount of the pharmaceutical composition to the patient if the patient suffers from ASD phenotype 3,
wherein it is determined that the patient suffers from ASD phenotype 3 if he shows at least two of the following first group of clinical signs and symptoms comprising macrocephaly, tachycardia, long neck, hypophosphatemia, McCune-Albright syndrome, seizures, hypertension, hyperkalemia, gastroesophageal reflux, precocious puberty, hyperinsulinemia, increased inflammatory response, familial history of cholangiocarcinoma, hearing impairment, growth hormone excess, tall stature, hyperchloraemia and type II diabetes.

6. Pharmaceutical composition for use according to any of claims 1 to 5, wherein the excitable cells are neuronal cells and beta pancreatic cells.

7. Pharmaceutical composition for use according to any of claims 1 to 6, wherein the substance capable of inducing repolarization of the membrane of excitable cells after depolarization is selected from the group of sulfanilamides, in particular azosemide, benzmetanide, clofenamide, clopamide, clorexolone, furosemide, indapamide, mefruside, metolazone, piretanide, torasemide (torsemide) and xipamide; meta-aminobenzoates, in particular bumetanide; benzothiadiazines, in particular bendroflumethiazide, chlorothiazide, cyclopenthiazide, cyclothiazide, hydrochlorothiazide, hydroflumethiazide, mebutizide, methylclothiazide, polythiazide and trichlormethiazide; thiazoles, in particular etozolin, ozolinone and tizolemide; quinazolines, in particular fenquizone and quinethazone; phthalimides, in particular chlortalidone; phenoxyacetates, in particular etacrynic acid; indans, in particular indocrinone; pyrazoles, in particular muzomimine; and indoles, in particular tripamide.

8. Pharmaceutical composition for use according to any of claims 1 to 7, wherein the substance capable of inducing repolarization of the membrane of excitable cells after depolarization is a derivative of 4-substituted-3-amino-5-sulfamoylbenzoic acid.

9. Pharmaceutical composition for use according to claim 8, wherein the derivative of 4-substituted-3-amino-5-sulfamoylbenzoic acid is selected from the group consisting of bumetanide, AqB007, AqB011, PF-2178, BUM13, BUM5, bumepamine and mixtures thereof.

10. Pharmaceutical composition for use according to any of claims 2 to 9, wherein the patient shows hyperinsulinemia and at least one other of the clinical signs and symptoms.

11. Pharmaceutical composition for use according to claim 2 to 10, wherein the patient shows at least three of first group of clinical signs and symptoms.

12. Pharmaceutical composition for use according to any of claims 1 to 10, wherein the substance capable of inducing repolarization of the membrane of excitable cells after depolarization is administered orally to the patient at a daily total dosage of between 0.01 to 10 mg total per day, preferably between 0.5 and 4 mg total a day.

13. Method for dividing an ASD patient cohort into patients responding to treatment with a substance capable of inducing repolarization of membrane of excitable cells after depolarization and patients not responding to said treatment, comprising the steps of:
- providing a patient database including data sets relating to the following first group of clinical signs and symptoms comprising macrocephaly, tachycardia, long neck, hypophosphatemia, McCune-Albright syndrome, seizures, hypertension, hyperkalemia, gastroesophageal reflux, precocious puberty, hyperinsulinemia, increased inflammatory response, familial history of cholangiocarcinoma, hearing impairment, growth hormone excess, tall stature, hyperchloraemia and type II diabetes; and
- classifying a patient as a patient responding to treatment with a substance capable of inducing repolarization of membrane of excitable cells after depolarization if he shows at least two of the clinical signs and symptoms of said first group.

14. Method for diagnosing an ASD patient with ASD phenotype 3, comprising the steps of:
- providing a patient-specific data set relating to the following first group of clinical signs and symptoms comprising macrocephaly, tachycardia, long neck, hypophosphatemia, McCune-Albright syndrome, seizures, hypertension, hyperkalemia, gastroesophageal reflux, precocious puberty, hyperinsulinemia, increased inflammatory response, familial history of cholangiocarcinoma, hearing impairment, growth hormone excess, tall stature, hyperchloraemia and type II diabetes; and
- diagnosing the patient with ASD phenotype 3 if he shows at least two of the clinical signs and symptoms of said first group.

15. Method according to claim 13 or 14, wherein the ASD patient is diagnosed with ASD phenotype 3 or classified as a patient responding to treatment with a substance capable of inducing repolarization of the membrane of excitable cells after depolarization if the patient shows hyperinsulinemia and at least one other of the clinical signs and symptoms of said first group.

16. Method according to any of claims 13 to 15, wherein the patient shows at least three of the clinical signs and symptoms of said first group.
